Europäisches Patentamt

European Patent Office

Office européen des brevets

(11) Veröffentlichungsnummer: **0 008 425**
**B1**

(12) **EUROPÄISCHE PATENTSCHRIFT**

(45) Veröffentlichungstag der Patentschrift:
07.07.82

(51) Int. Cl.³: **G 01 N 5/04,** B 01 D 37/00,
G 01 N 33/24

(21) Anmeldenummer: **79102905.1**

(22) Anmeldetag: **10.08.79**

(54) Verfahren zur Bestimmung des Entwässerungsverhaltens von Schlammproben.

(30) Priorität: 24.08.78 DE 2837017

(43) Veröffentlichungstag der Anmeldung:
05.03.80 Patentblatt 80/5

(45) Bekanntmachung des Hinweises auf die Patenterteilung:
07.07.82 Patentblatt 82/27

(84) Benannte Vertragsstaaten:
AT CH DE FR SE

(56) Entgegenhaltungen:
DE-A-1 916 701

SLUDGE DEWATERING, Manual of Practice, Nr. 20, 1970, Seiten 26–36 Ausg. Water Pollution Control Federation Washington, D.C., USA «2.5 The sludge conditioning program»

JOURNAL OF THE WATER POLLUTION CONTROL FEDERATION, Vol. 50, Nr. 8, August 1978, Seiten 1965–1975 Washington, D.C., USA D. ZENZ et al.: «Evaluation of dewatering equipment for anaerobically digested sludge»

(73) Patentinhaber: BASF Aktiengesellschaft,
Carl-Bosch-Strasse 38, D-6700 Ludwigshafen (DE)

(72) Erfinder: Sander, Bruno, Dr., Moerikestrasse 10,
D-6700 Ludwigshafen (DE)

JOURNAL OF THE WATER POLLUTION CONTROL FEDERATION, Vol. 46, 1974 Seiten 1914–1926 Washington, D.C., USA J.C. O'SHAUGHNESSY et al.: «Digestion and dewatering of phosphorus-enriched sludges»

Verfahren zur Bestimmung des Entwässerungsverhaltens von Schlammproben

Die Erfindung betrifft ein Verfahren zur Bestimmung des Entwässerungsverhaltens von Schlämmen durch Ermittlung der Filtratmengen in bestimmten Zeitintervallen und der Feststoffgehalte in den Filterkuchen mittels Entwässerung der Schlammprobe durch Anlagen einer Druckdifferenz zu dem Zweck der Auswahl geeigneter Entwässerungshilfsmittel nach Art und Menge, der Auswahl einer geeigneten Entwässerungsmaschine und der Voraussage der unter technischen Bedingungen erzielbaren Entwässerungseffekte.

Zur Charakterisierung des Entwässerungsverhaltens von Schlämmen sind verschiedene Möglichkeiten bereits bekannt. So gibt die Bestimmung des spezifischen Filterwiderstandes eines Klärschlammes Hinweise für seine Entwässerungsfähigkeit in einer Kammerfilterpresse. Diese Methode ist messtechnisch und auswertetechnisch kompliziert und zeitaufwendig. Die erhaltenen Werte können nicht auf andere Entwässerungsmaschinen wie z.B. Dekanter oder Siebbandpressen übertragen werden.

Für die Ermittlung der Zugabemengen verschiedener Flockungsmittel zur Verbesserung der Filtrierbarkeit von Schlämmen ist eine Labormethode bekannt, bei der die Schlammprobe auf einem Büchnertrichter aufgegeben und unmittelbar ein Wasserstrahlvakuum angelegt wird. Als Filtermedium wird Filterpapier benutzt. Aufgrund der pro Zeiteinheit anfallenden Filtratmenge kann das wirksamste Flockungsmittel ermittelt werden. Das Ende des Filtrationsvorganges ist durch den Zusammenbruch des Vakuums infolge Rissbildung im Filterkuchen gegeben. Da viele Schlämme beim Entwässern zur Rissbildung neigen, ist diese Methode nur begrenzt anwendbar.

Nach einer anderen Methode zur Bestimmung des Entwässerungsverhaltens von Klärschlämmen wird die kapillare Fliesszeit des aus dem Schlamm freigesetzten Schlammfiltrats in einem definierten Filterpapier bestimmt. Bei diesem Messverfahren handelt es sich um eine indirekte Bestimmungsmethode, da erst Beziehungen zwischen den im Labor ermittelten Kennwerten und den jeweiligen Daten des technischen Betriebes hergestellt werden müssen. Ausserdem ist diese Methode nicht allgemein anwendbar, da bei der Zugabe des Flockungsmittels eine Rührerdrehzahl von 1000 U./min empfohlen wird. So werden z.B. geflockte Flotationsbergeschlämme aus dem Steinkohlenbergbau, die sehr gut geflockt und auf Siebbandpressen mit gutem Ergebnis entwässert werden können, bei Rührerdrehzahlen von grösser als 100 U./min zerstört und die Entwässerungsfähigkeit nachteilig beeinflusst.

Aus der DE-A-1 916 701 ist ein Verfahren zum Entwässern von Abwasserschlamm bekannt, bei dem eine kontinuierliche zweistufige Entwässerung durch Schwerkraftfiltration und anschliessend durch Druckfiltration im technischen Massstab ausgeführt wird. Mit dieser Methode kann keine umfassende Charakterisierung des Entwässerungsverhaltens von Schlämmen unabhängig von der jeweils verwendeten Entwässerungsvorrichtung erfolgen.

Zum besseren Verständnis der vorliegenden Erfindung soll noch erwähnt werden, dass die Auswahl einer geeigneten Entwässerungsmaschine, wie z.B. Kammerfilterpresse, Dekanter oder Siebbandpresse, und die Erprobung geeigneter Entwässerungshilfsmittel in den meisten Fällen erst im technischen Massstab auf diesen technologisch so grundsätzlich verschiedenen Maschinen erfolgen. Ein solches rein empirisches Vorgehen ist zeitraubend und kostenintensiv.

Aufgabe der Erfindung war es demnach, ein Messverfahren zur Bestimmung des Entwässerungsverhaltens von Schlämmen zu erarbeiten, bei dem durch bestimmte Massnahmen eine sichere Auswahl der geeigneten Entwässerungshilfsmittel (z.B. Flockungsmittel) nach Art und Menge getroffen werden kann, die vorherige Auswahl der am besten geeigneten Entwässerungsmaschine ermöglicht wird und Voraussagen über die im technischen Massstab erzielbaren Entwässerungseffekte gemacht werden können. Das erfindungsgemässe Verfahren soll allgemein auf möglichst alle Schlämme anwendbar sein und einen unmittelbaren Bezug zum technischen Entwässerungsvorgang haben.

Diese Aufgabe wird erfindungsgemäss dadurch gelöst, dass die Schlammprobe in einem ersten Messschritt bis zu 30 Minuten lang einer Schwerkraftfiltration unterworfen wird und dieselbe bereits vorentwässerte Schlammprobe in einem zweiten Messschritt durch Anlegen eines Vakuums von 400 mbar über eine Zeitdauer von 10 Minuten oder durch Anwendung von Pressdrücken von 1 bis 10 bar, vorzugsweise 5 bis 8 bar, über eine Zeitdauer von 1 bis 20 Minuten, vorzugsweise von 2 bis 3 Minuten, weiterhin entwässert wird. Die in den Presskuchen erhaltenen Feststoffgehalte geben unmittelbar Auskunft über die im technischen Massstab erzielbaren Entwässerungseffekte.

Die Durchführung des erfindungsgemässen Verfahrens wird nachfolgend in einer bevorzugten Ausführung rein beispielsweise beschrieben:

Ausführungsform A

1. Probenvorbehandlung

500 ml einer Schlammprobe werden in ein 1-Liter-Becherglas (hohe Form) gegeben und unter Rühren bei einer Drehzahl von 50 U./min innerhalb von 20 Sekunden mit den Entwässerungshilfsmitteln, z.B. einer frisch zubereiteten 0,1%igen wässrigen Flockungsmittel-Lösung, versetzt. Es wird ein quadratischer Blattrührer (70 mm) mit 6 quadratischen Aussparungen (10 mm) verwendet. Die Rührzeit soll 2 Minuten betragen. Bei Reihenversuchen mit verschiedenen Entwässerungshilfsmitteln sowie bei der unbehandelten Kontrollprobe (Nullprobe) muss durch Zugabe berechneter Wassermengen dafür gesorgt werden, dass das Gesamtvolumen der Proben und damit die Konzentration der Schlammfeststoffe konstant ist.

## 2. Schwerkraftfiltration (Messschritt I)

Die vorbehandelte Schlammprobe wird auf eine Büchner-Nutsche aus Duranglas mit plangeschliffenem Schlitzsieb (110 mm$\emptyset$) gegeben, auf dem ein Filtergewebe aus Polypropylen-Leinen (110 mm$\emptyset$) gelegt wurde. Das ablaufende Filtrat wird in einem Messzylinder (500 ml) aufgefangen. Nach 5, 10, 20 und 30 Minuten wird die Filtratmenge (ml) gemessen. Die nach 30 Minuten erhaltene Filtratmenge gibt Aufschluss über die Schwerkraft-Entwässerungsfähigkeit eines Schlammes.

## 3. Vakuumfiltration (Messschritt II)

Die Büchner-Nutsche mit der vorentwässerten Schlammprobe wird auf eine vakuumfeste Saugflasche (500 ml) aufgesetzt und durch Anlegen eines Vakuums von 400 mbar weiterhin entwässert. Nach jeweils 5 und 10 Minuten wird die Filtratmenge in ml gemessen. Die nach 10 Minuten erhaltene Filtratmenge gibt Aufschluss über die Entwässerungsfähigkeit eines Schlammes beim Anlegen einer Druckdifferenz in einer 2. Entwässerungsstufe.

Die Trockenstoff-Gehalte TS (%) der Filterkuchen nach 30 Minuten Schwerkraftfiltration und nach 10 Minuten zusätzlicher Vakuumfiltration werden aus dem gravimetrisch bestimmten Trockenstoff-Gehalt der ursprünglichen Schlammprobe und den erhaltenen Filtratmengen berechnet. Dabei muss die zur Einstellung des Gesamtvolumens erforderliche Wassermenge berücksichtigt werden. Die Restfeuchte des Filterkuchens kann zusätzlich gravimetrisch bestimmt werden.

## Ausführungsform B

Die Probenvorbehandlung und der Messschritt I (Schwerkraftfiltration) erfolgen wie in der Ausführungsform A beschrieben.

## Druckfiltration (Messschritt II)

Der vorentwässerte Klärschlamm wird in einer Stempelpresse (Fabrikat: Hubert, Sneek/Holland) einer Druckfiltration in der Dünnschicht unterworfen. Die Pressplatte des Stempels ist quadratisch (Kantenlänge: 220 mm). Die Bodenplatte der Stempelpresse ist als Filterplatte ausgebildet. Auf diese Filterplatte wird ein Filtergewebe aus Polypropylen-Leinen gelegt. Für die seitliche Begrenzung wird noch ein Schaumgummi-Kragen (Höhe: 40 mm) mit einer Aussparung von 145 × 145 mm aufgesetzt. Hierdurch wird der Schlamm am seitlichen Austreten gehindert. In dieser Aussparung wird der vorentwässerte Schlamm gleichmässig verteilt. Danach wird der Stempel der Presse langsam nach unten geführt. Der Pressdruck wird allmählich innerhalb von 1 bis 3 Minuten bis auf 10 bar gesteigert. Der Enddruck von 10 bar wird 2 Minuten lang aufrecht gehalten. Das freigesetzte Wasser läuft durch das Filtertuch nach unten und seitlich durch den Schaumgummi-Kragen ab. Der erhaltene Presskuchen wird vermessen und analysiert. Die Schichtdicken der Presskuchen liegen je nach Schlammart und Feststoffkonzentration der Schlämme zwischen 2 und 5 mm. Die Restfeuchte der Presskuchen wird gravimetrisch bestimmt. Mit diesen Ausführungsformen der Erfindung kann das Entwässerungsverhalten der meisten Schlämme ausreichend beschrieben werden und es können die gestellten Aufgaben gelöst werden.

In Sonderfällen kann das erfindungsgemässe Verfahren abgewandelt werden: Bei der Probenvorbehandlung können die Rührer-Drehzahlen bis auf 100 U./min gesteigert werden. Höhere Drehzahlen sollten jedoch vermieden werden, sofern es sich nicht um mechanisch besonders stabile Schlämme handelt. Anstelle des Blattrührers kann z.B. auch ein Balken- oder ein Gitterrührer eingesetzt werden. Die Vermischung der Schlammproben mit den Entwässerungshilfsmitteln kann auch in einem 1-Liter-Mischzylinder durch z.B. zehnmaliges Umstürzen innerhalb von 30 Sekunden erfolgen. Diese Methode eignet sich besonders bei mechanisch instabilen Schlämmen. Unabhängig von der Art der Probenvorbehandlung soll eine Kontaktzeit der Schlammprobe und der Entwässerungshilfsmittel von mindestens 2 Minuten eingehalten werden, bevor der Messschritt I begonnen wird.

Bei den Filtrationen wird als Filtermaterial ein Filtergewebe aus Polypropylen-Leinen mit einem Flächengewicht von 290 g/m² (Hersteller: Walraf Textilwerke, Mönchengladbach 2) verwendet, das den auf Entwässerungsmaschinen eingesetzten Filtergeweben (Bänder, Tücher) sehr ähnlich ist. Bei den bekannten Verfahren werden dagegen, wie eingangs ausgeführt, definierte Filterpapiere benutzt. Mit dem erfindungsgemässen Verfahren können demnach auch verschiedene technische Filtergewebe auf ihre Brauchbarkeit geprüft werden. Hierbei kann neben dem Filtratanfall auch ein eventueller Feststoff-Durchschlag ermittelt werden. Ebenso können Hinweise über das Ablöseverhalten der Filterkuchen vom Filtergewebe erhalten werden.

Die Filterplatten der verwendeten Büchner-Nutschen aus Glas sollen plangeschliffen sein, damit das Filtergewebe eng aufliegt. Bei den üblichen Büchnernutschen aus Porzellan stören die Wölbungen der gelochten Filterplatte.

Zur Erzeugung des Vakuums kann eine Wasserstrahlpumpe oder eine Drehschieberpumpe (z.B. der Firma Leybold-Heraeus) verwendet werden. Die Reduzierung des Vakuums auf 400 mbar geschieht auf die übliche Weise. Das geringe Vakuum wird angewandt, damit keine Rissbildung erfolgt und möglichst viele Schlämme der Messung zugänglich werden. Auf diese Weise kann der Filtratanfall nach bestimmten Zeitintervallen ermittelt und es können grafisch echte Funktionen dargestellt werden.

Für die Durchführung der Druckfiltration (Ausführungsform B, Messschritt II) können auch andere Stempelpressen eingesetzt werden, mit denen die erfindungsgemässen Pressbedingungen eingehalten werden können.

Die Pressdrücke und Presszeiten können innerhalb weiter Grenzen schwanken und den Betriebsbedingungen spezieller Entwässerungsmaschinen angepasst werden. So können z.B. für eine Siebbandpresse vom Langzeittyp, bei der relativ geringe reale Pressdrücke von z.B. 0,8 bis 1,2 bar und lange Presszeiten bis zu 20 Minuten angewandt werden, die entsprechenden Pressbedingungen nachgestellt werden. Dies gilt gleichermassen auch für eine Siebbandpresse vom Kurzzeittyp, bei der relativ hohe reale Pressdrücke bis 5 bar und kurze Presszeiten von 2,5 bis 4 Minuten üblich sind.

Für die Beschreibung des Entwässerungsverhaltens von Schlämmen haben sich jedoch für die Druckfiltration folgende Pressbedingungen bewährt: Anwendung eines Pressdruckes von 10 bar (pneumatischer Druck des Druckluftzylinders, entsprechend einem realen Pressdruck auf das Pressgut von 8 bar) und einer Presszeit von 2 Minuten.

Als Entwässerungshilfsmittel werden synthetische, organische Flockungsmittel, die sogenannten Polyelektrolyte eingesetzt. Diese werden in Form ihrer 0,05 bis 1,0 und bevorzugt 0,1 bis 0,3prozentigen wässrigen Lösungen verwendet. Ferner können wässrige Lösungen anorganischer Entwässerungshilfsmittel, wie z.B. der Eisen- und Aluminiumsalze, eingesetzt werden. In den meisten Fällen wird zusätzlich noch Kalkmilch zugesetzt, damit eine Flockung, Fällung oder Kollektorfällung der Schlammfeststoffe und der Schlammtrübstoffe (kolloidale Bestandteile) bewirkt wird. Selbstverständlich können den Schlämmen auch verschiedene Zusätze wie z.B. feinteilige Kohle, Asche, Bentonit, Sägemehl, Steinmehl, Dolomit usw. zugeschlagen werden.

Ein besonderer Vorteil des erfindungsgemässen Verfahrens besteht darin, dass ausser der Entwässerungsfähigkeit eines Schlammes bei Schwerkraftfiltration insbesondere auch das Entwässerungsverhalten beim Anlegen einer Druckdifferenz in einem zweiten Verfahrensschritt bestimmt wird. Auf diese Weise werden häufig auftretende Fehlbeurteilungen vermieden. Es gibt Entwässerungshilfsmittel oder Entwässerungshilfsmittel-Kombinationen, die zwar eine gute Schwerkraft-Entwässerungsfähigkeit hervorrufen, jedoch beim Anlegen einer Druckdifferenz einen nur mittelmässigen Entwässerungseffekt liefern. Auch das umgekehrte Entwässerungsverhalten kann häufig festgestellt werden. Erst aus der Ermittlung und Darstellung des gesamten Entwässerungsverhaltens kann sicher geschlossen werden, ob eine Entwässerung einstufig unter Druck, z.B. auf einer Kammerfilterpresse, einstufig durch Vakuumfiltration oder zweistufig durch Schwerkraftfiltration und nachfolgender Druckfiltration, z.B. auf einer Siebbandpresse durchgeführt werden soll. Mit dem erfindungsgemässen Verfahren wird also eine umfassende entwässerungstechnische Charakterisierung eines Schlammes erreicht.

Ein wirtschaftlicher Vorteil des erfindungsgemässen Verfahrens besteht weiterhin darin, dass die Optimierung der zu verwendenden Entwässerungshilfsmittel je nach der beabsichtigten Art der technischen Entwässerung stoffspezifisch und unabhängig von der besonderen Ausführungsform einer Maschine (Fabrikat) bereits in dem kostengünstigeren Labormassstab vorgenommen werden kann. Aufwendige Untersuchungen auf verschiedenartigen Entwässerungsmaschinen unter Anwendung verschiedener Entwässerungshilfsmittel über längere Zeiträume sind nicht mehr erforderlich.

Ein weiterer Vorteil des erfindungsgemässen Verfahrens ist dadurch gegeben, dass einerseits die laufende Überwachung der Entwässerungseigenschaften eines Schlammes unabhängig von einer vorgegebenen Entwässerungsmaschine erfolgen kann und anderseits auch die technische Funktionsfähigkeit einer Entwässerungsmaschine und deren Verfahrensparameter überprüft werden können.

Die Erfindung wird nachfolgend anhand von Beispielen näher erläutert. Die dort angegebenen Prozentzahlen sind immer Gew.-%.

Beispiel 1

Der Überschussschlamm einer biologischen Kläranlage eines pharmazeutischen Unternehmens mit einem Feststoffgehalt von 5,0% und einem Gehalt an organischen Bestandteilen im Feststoff von 44,5% wurde mit den verschiedensten organischen Flockungsmitteln behandelt und danach das Entwässerungsverhalten nach Ausführungsform A bestimmt. In diesem Fall lieferten kationische Flockungsmittel die günstigsten Entwässerungsergebnisse und wurden für die weitere Untersuchung ausgewählt.

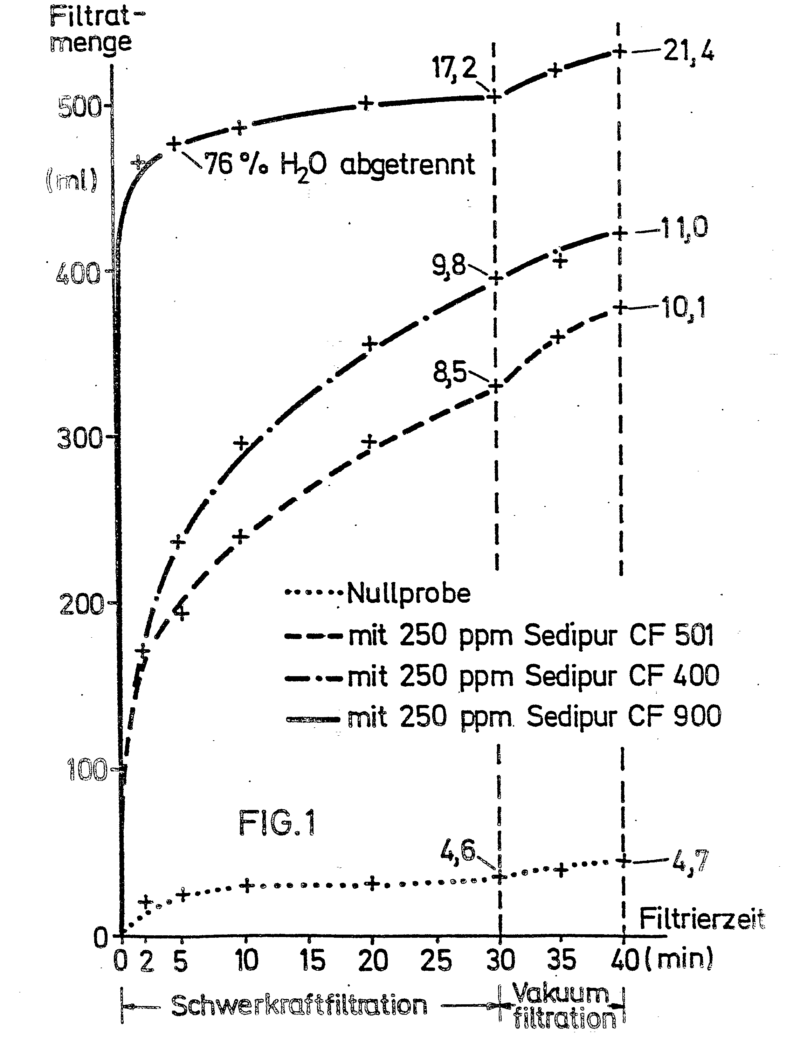

Das Entwässerungsverhalten und die erzielten Ergebnisse der mit den kationischen Flockungsmitteln ®Sedipur* CF 900, CF 501 und CF 400 behandelten Proben sowie der unbehandelten Probe sind in Fig. 1 dargestellt. Bereits nach 5minutiger Schwerkraftfiltration können mit dem wirksamsten Flockungsmittel ca. 76% des durch die Flockreaktion verfügbar gewordenen Wassers abgetrennt werden. Durch Anlegen einer Druckdifferenz wurde eine weitere Steigerung der Feststoffgehalte in den Filterkuchen erreicht, vgl. die Zahlenangaben bei den Kurven nach 30 Minuten und nach 40 Minuten Entwässerungsdauer.

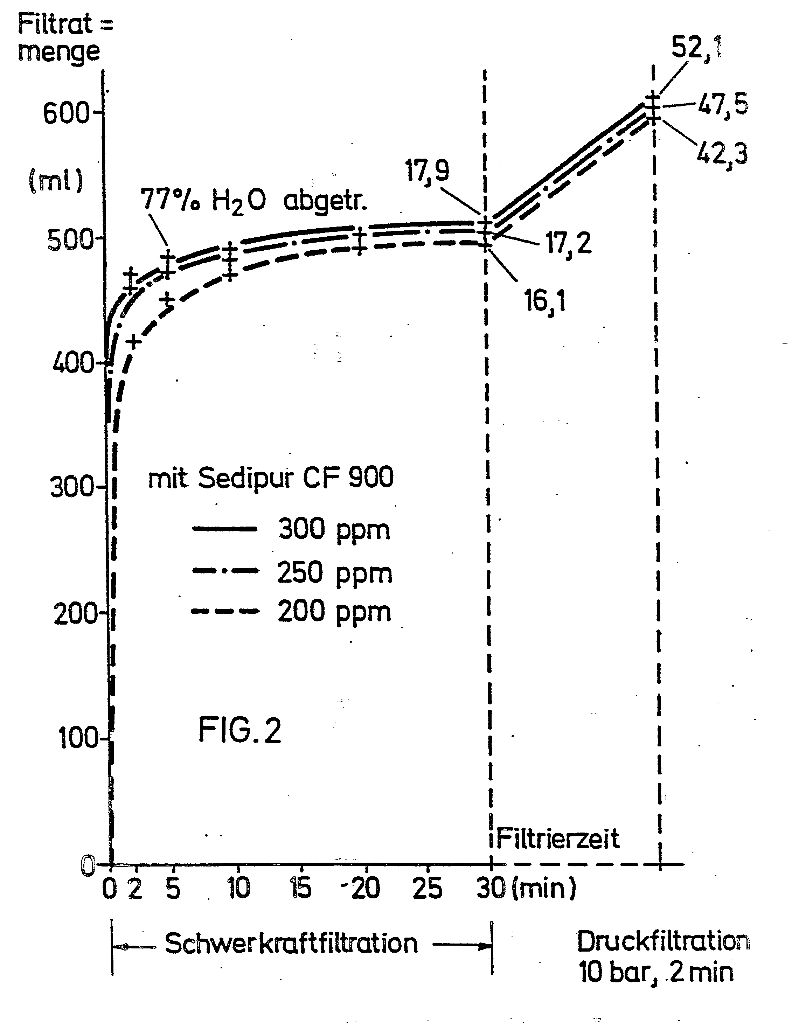

Zur Optimierung der erforderlichen Flockungsmittelmenge wurde nach der Ausführungsform B verfahren. Die erhaltenen Ergebnisse gehen aus Fig. 2 hervor. Nach den erhaltenen Ergebnissen mit verschiedenen Flockungsmittelmengen muss eine zweistufige Entwässerung in der Dünnschicht, die z.B. auf einer Siebbandpresse ausgeführt werden kann, unter Anwendung von 300 ppm (= 300 g/m³ Schlamm bei 5% Feststoffgehalt) des Flockungsmittels® Sedipur CF 900 empfohlen werden.

---

\* ®Sedipur: eingetragenes Warenzeichen der Firma BASF AG

**Beispiel 2**

Die nachfolgenden Untersuchungen wurden an einem Vorklärschlamm durchgeführt, der in der Vorklärstufe einer Kläranlage eines chemischen Unternehmens durch Sedimentation erhalten worden ist. Der Schlamm besass einen Feststoffgehalt von 2,9%, und der Anteil an organischen Bestandteilen der Schlammfeststoffe betrug 50,0%.

Die Auswahl des wirksamsten Flockungsmittels und der optimalen Menge erfolgte nach der Ausführungsform A. Ein anionisches Flockungsmittel (®Sedipur AF 400) erwies sich als das günstigste Flockungsmittel in einer Menge von 100 ppm (g/m³ Schlamm).

Zum Vergleich wurden einer Schlammprobe von 500 ml auch übliche anorganische Entwässerungshilfsmittel zugesetzt. Bei der Probenvorbehandlung wurden nach dem Prinzip der Dreifachkonditionierung 5,8 g Klärschlammasche, 2,9 g $FeSO_4$ in Form einer 20%igen wässrigen Lösung und 5,8 g $Ca(OH)_2$ in Form einer 20%igen wässrigen Suspension (Kalkmilch) in der angegebenen Reihenfolge zugesetzt. Die Rührer-Drehzahl musste auf 200 U./min erhöht werden. Die Rührzeit betrug nach Zugabe der Kalkmilch-Suspension 2 Minuten.

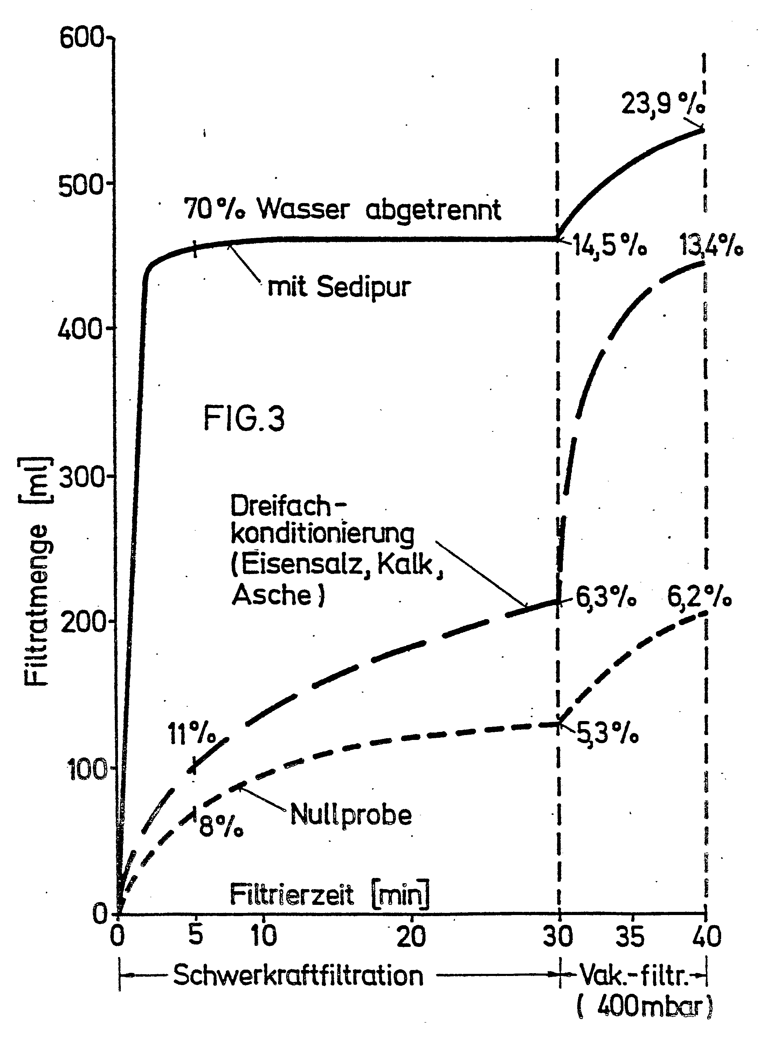

Die mit organischen und mit anorganischen Entwässerungshilfsmitteln vorbehandelten Proben sowie eine unbehandelte wurden der Ausführungsform A unterworfen. Deren Entwässerungsverläufe und die erhaltenen Ergebnisse gehen aus Fig. 3 hervor.

Bei der mit anorganischen Entwässerungshilfsmitteln behandelten Schlammprobe wird die Schwerkraftentwässerungsfähigkeit gegenüber der unbehandelten nur unwesentlich verbessert. Die Hauptentwässerung findet erst beim Anlegen einer Druckdifferenz statt. Die technische Entwässerung sollte in diesem Falle einstufig z.B. in einer Kammerfilterpresse vorgenommen werden. Die in Fig. 3 angegebenen Trockenstoffgehalte beziehen sich nur auf den Gehalt an Schlammtrockenstoff. Die Zuschlagstoffe wurden wegen der Vergleichbarkeit nicht berücksichtigt. Ein grundsätzlich andersartiges Entwässerungsverhalten zeigt die mit einem organischen Flockungsmittel behandelte Schlammprobe. Hier können bereits nach 5minutiger Schwerkraftfiltration 70% des Wassers abgetrennt werden. Beim Anlegen einer Druckdifferenz werden dann weit höhere Feststoffgehalte erzielt als bei der Verwendung anorganischer Entwässerungshilfsmittel. In diesem Fall muss eine zweistufige Entwässerung in der Dünnschicht empfohlen werden. Der Vorklärschlamm wurde demzufolge auf einer Siebbandpresse der Firma Alfa-Laval (Bandbreite: 100 cm) zweistufig entwässert. Die nachfolgende Tabelle 1 enthält einen Vergleich der nach Ausführungsform B des erfindungsgemässen Verfahrens erhaltenen Daten mit den unter technischen Bedingungen tatsächlich erzielten Daten:

Tabelle 1

|  | Labor-daten | Maschinen-daten |
|---|---|---|
| Flockungsmittelverbrauch: |  |  |
|   g/m³ | 120 | 132 |
|   kg/t TS | 2,5 | 2,6 |
| Trockenstoffgehalt der Filterkuchen (%) |  |  |
| nach Schwerkraftfiltration | 12,3 | 12,3 |
| nach Druckfiltration | 39,8 | 39,8 |

**Beispiel 3**

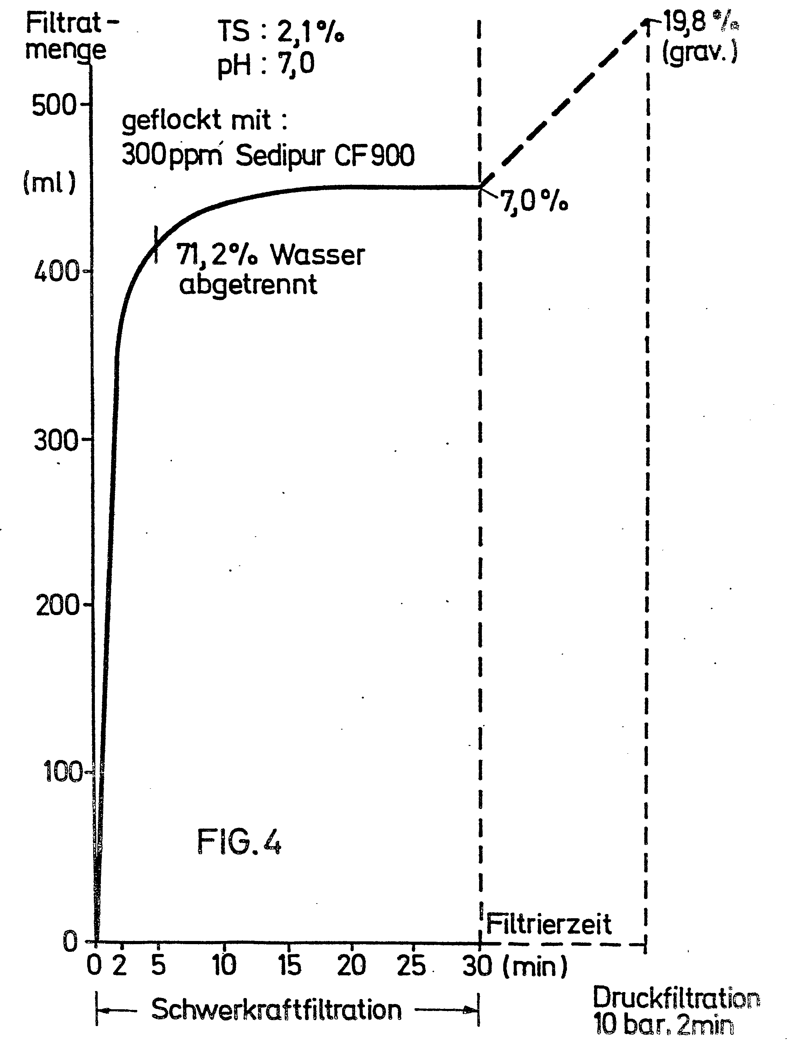

Ein durch Langzeitbelüftung bei Umgebungstemperatur mineralisierter Schlamm einer Versuchskläranlage eines chemischen Unternehmens wurde mit verschiedenen organischen Flockungsmitteln versetzt und das Entwässerungsverhalten nach den Methoden der Ausführungsform A und B bestimmt. Der Schlamm besass einen Trockenstoffgehalt von 2,1%, war feinteilig und durch einen hohen Gehalt an Zellwasser sehr schwer entwässerbar. Das günstigste Entwässerungsergebnis wird in Fig. 4 wiedergegeben. Hiernach können bei Flockung mit 300 g ®Sedipur CF 900/m³ Schlamm nach der Schwerkraftfiltration etwa 7% und nach der Druckfiltration 19 bis 20% Feststoffgehalt in dem Filterkuchen erwartet werden.

Es wurden 7 verschiedene Fabrikate von Siebbandpressen (= SBP), die in den Verfahrensstufen Schlammvorbehandlung (Flockung), Vorentwässerung (Schwerkraftfiltration) und Pressung (Druckfiltration) technisch relevante Unterschiede aufwiesen, auf ihre Wirksamkeit bei der Entwässerung des mineralisierten Schlamms überprüft. Die erzielten Ergebnisse gehen aus Tabelle 2 hervor.

Nur die SBP 7 zeigte gegenüber dem Labortest einen erhöhten Flockungsmittelverbrauch, was auf eine unzureichende Art der Flockung zurückgeführt wurde. Die Schlammtrockenstoffgehalte gemäss Labortest-Voraussage konnten nach Schwerkraftfiltration von SBP 5 und 7 und nach Druckfiltration von SBP 1 und 5 erreicht werden.

Dieses Beispiel zeigt die Bedeutung des erfindungsgemässen Verfahrens für die Praxis, sowohl für den Betreiber als auch für den Maschinenbauer.

Tabelle 2

| | Flockungsmittel-Verbrauch | | Schlammtrockenstoff-Gehalt (%) | | |
| | g/m³ | kg/t TS | Ausgangs-konzentration | nach Schwer-kraftfiltration | nach Druck-filtration |
|---|---|---|---|---|---|
| Labortest | 300 | 13,6 | 2,2 | 7,0 | 19–20 |
| SBP 1 | 300 | 15,8 | 1,9 | – | 20,4 |
| SBP 2 | 300 | 14,3 | 2,1 | 6,4 | 15,0 |
| SBp 3 | 320 | 15,2 | 2,1 | 5,9 | 15,7 |
| SBP 4 | 295 | 13,4 | 2,2 | 6,0 | 14,8 |
| SBP 5 | 312 | 13,6 | 2,3 | 6,8 | 19,3 |
| SBP 6 | 290 | 13,2 | 2,2 | 3,9 | 18,0 |
| SBP 7 | 370 | 16,8 | 2,2 | 6,8 | 14,5 |

Beispiel 4

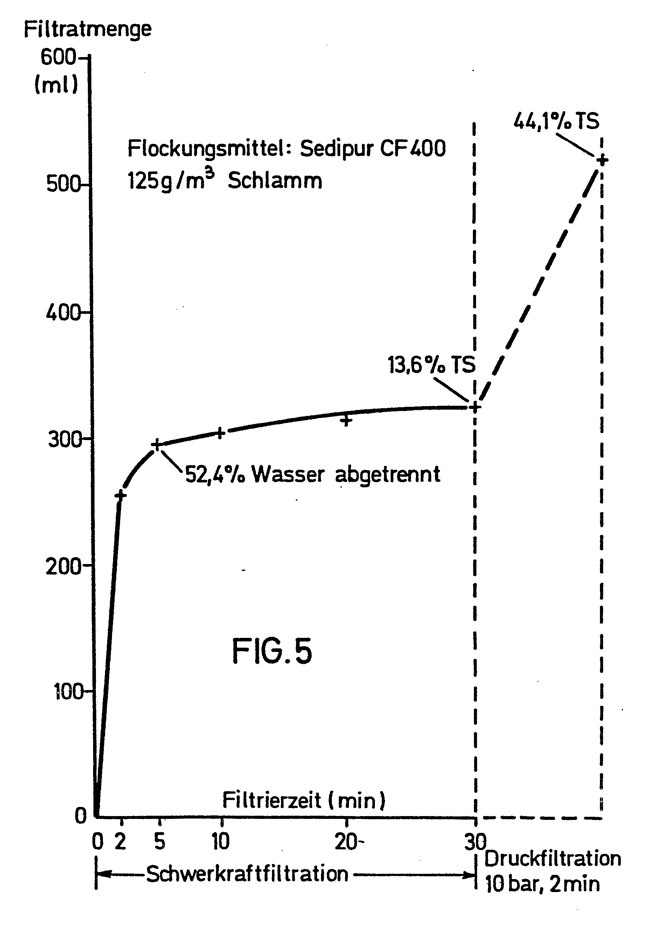

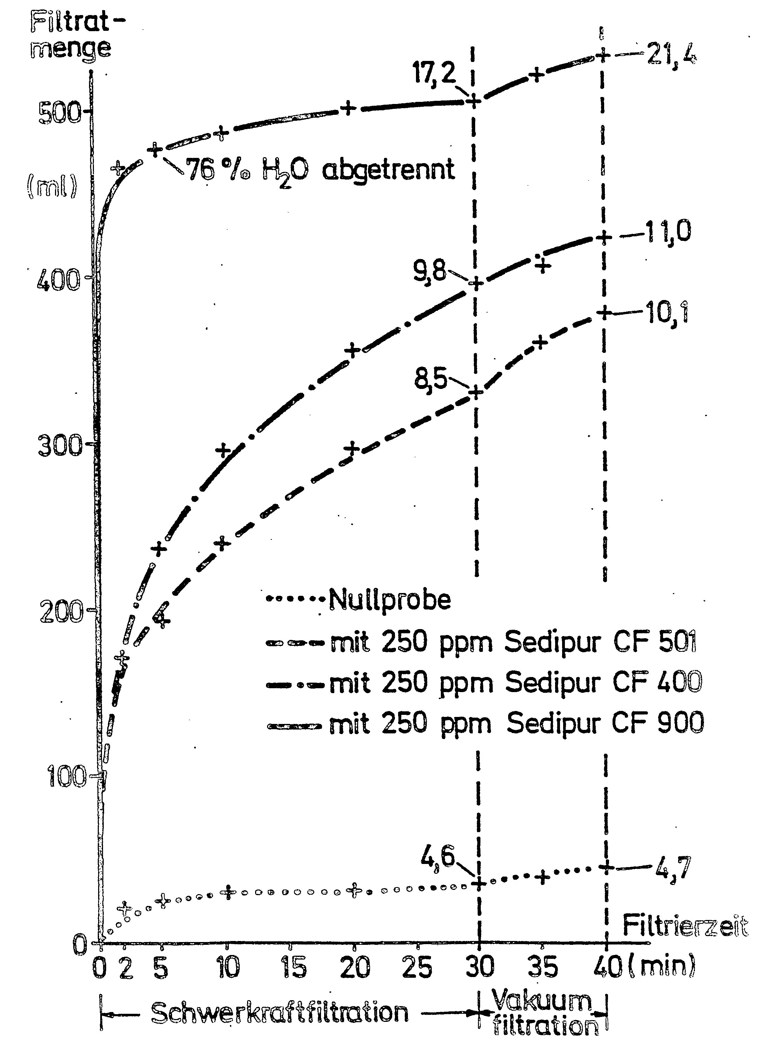

Der Faulschlamm einer kommunalen Kläranlage mit einem Feststoffgehalt von 7,5% und einem Anteil an organischen Bestandteilen am Schlammfeststoff von 37,9% sollte maschinell entwässert werden. Die Auswahl eines geeigneten Flockungsmittels erfolgte nach Ausführungsform A und B. Es zeigte sich, dass ein kationisches Flockungsmittel mit 40%iger kationischer Modifizierung die besten Entwässerungsergebnisse bei der zweistufigen Entwässerung lieferte. Die Ergebnisse zeigte Fig. 5. Einen Vergleich der Daten des Labortests nach Ausführungsform B mit den Entwässerungsergebnissen, die mit einer Siebbandpresse der Firma von Roll mit einer Bandbreite von 100 cm erzielt worden sind, zeigt die nachfolgende Tabelle 3.

Tabelle 3

| | Labor-daten | Maschinen-daten |
|---|---|---|
| Flockungsmittelverbrauch g/m³ Schlamm | 125 | 125 |
| Trockenstoffgehalt der Filterkuchen (%) | | |
| nach Schwerkraftfiltration | 13,6 | 13,6 |
| nach Druckfiltration | 44,1 | 43,7 |

**Patentansprüche**

Verfahren zur Bestimmung des Entwässerungsverhaltens von Schlammproben im Labormassstab durch Ermittlung der Filtratmengen in bestimmten Zeitintervallen und der Feststoffgehalte in den Filterkuchen mittels Entwässerung der Schlammprobe durch Anlegen einer Druckdifferenz, dadurch gekennzeichnet, dass die Schlammprobe in einem ersten Messschritt bis zu 30 Minuten lang einer Schwerkraftfiltration unterworfen wird und dieselbe bereits vorentwässerte Schlammprobe in einem zweiten Messschritt entweder durch Anlegen eines Vakuums von 400 mbar über eine Zeitdauer von 10 Minuten oder durch Anwendung von Pressdrücken von 1 bis 10 bar, vorzugsweise von 5 bis 8 bar, über eine Zeitdauer von 1 bis 20 Minuten, vorzugsweise von 2 bis 3 Minuten, weiterhin entwässert wird.

**Claims**

A method of determining the dewatering behaviour of sludge samples on a laboratory scale by determining the amounts of filtrate at specific intervals and the solids contents in the filter cakes by dewatering the sludge sample by applying a pressure difference, characterized in that, in a first measuring step, the sludge sample is subjected to gravity filtration for up to 30 minutes and, in a second measuring step, the same pre-dewatered sludge sample is further dewatered either by applying a vacuum of 400 mbar for a period of 10 minutes or by compression at pressures of from 1 to 10 bar, preferably from 5 to 8 bar, for a period of from 1 to 20 minutes, preferably from 2 to 3 minutes.

**Revendications**

Procédé de détermination du comportement à la dessication d'échantillons de boue, à l'échelle du laboratoire, par établissement des quantités de filtrat, à intervalles de temps déterminés, et des teneurs en matière solide dans le gâteau de filtre, au moyen d'une dessication de l'échantillon de boue par application d'une différence de pression, caractérisé par le fait que l'échantillon de boue est soumis, dans un premier stade de mesure, à une filtration par gravité, pendant une durée allant jusqu'à 30 minutes, et le même échantillon de boue déjà préséché est encore soumis à dessication, soit par application d'un vide de 400 mbars, pendant une durée de 10 minutes, soit par application de pressions de pressage de 1 à 10 bars, de préférence de 5 à 8 bars, pendant une durée de 1 à 20 minutes, de préférence de 2 à 3 minutes.

FIG.1

**Filtrat = menge (ml)**

77% H₂O abgetr.

mit Sedipur CF 900

───── 300 ppm

──·── 250 ppm

───── 200 ppm

FIG.2

17,9
17,2
16,1

52,1
47,5
42,3

Filtrierzeit

|← Schwerkraftfiltration →| Druckfiltration 10 bar, 2 min

0 2 5 10 15 20 25 30 (min)

0 008 425

FIG.3

Filtratmenge [ml] — vertical axis (0, 100, 200, 300, 400, 500, 600)

70% Wasser abgetrennt

mit Sedipur

23,9 %

14,5 %   13,4%

Dreifachkonditionierung
(Eisensalz, Kalk,
Asche )

6,3%   6,2%

11%

5,3%

8%   Nullprobe

Filtrierzeit [min]

0   5   10   20   30   35   40

├─ Schwerkraftfiltration ─┤ ├─ Vak.-filtr.-┤
( 400mbar )

11

0 008 425

Filtrat-menge

TS : 2,1 %
pH : 7,0

geflockt mit :
300 ppm Sedipur CF 900

↑19,8 %
(grav.)

7,0 %

71,2 % Wasser
abgetrennt

FIG. 4

500

400

300

200

100

0

(ml)

0 2  5    10   15   20   25   30 (min)

Filtrierzeit

Schwerkraftfiltration

Druckfiltration
10 bar. 2 min

13

Filtratmenge

FIG.5

Flockungsmittel: Sedipur CF 400 125g/m³ Schlamm

52,4% Wasser abgetrennt

13,6% TS

44,1% TS

Filtrierzeit (min)

Schwerkraftfiltration

Druckfiltration 10 bar, 2min